# EUROPEAN PATENT SPECIFICATION

(11) **EP 2 953 617 B1**
(45) Date of publication and mention of the grant of the patent: **02.10.2019**
(21) Application number: 14703341.9
(22) Date of filing: 06.02.2014
(51) Int. Cl.: A61K 9/16, A61K 36/77, A61K 45/06, A61K 47/12, A61K 47/14, A61K 31/167, A61K 31/375, A61K 31/4172, A61K 31/4415, A61K 31/485, A61K 31/495, A61K 31/522, A61K 31/675, A61K 31/7034, A61K 33/06, A61K 36/258, A61K 36/45, A61K 9/20, A61K 31/137, A61K 31/702, A61K 36/00

(54) **PHARMACEUTICAL COMPOSITIONS INCORPORATING LOW-DOSE DRUGS**
PHARMAZEUTISCHE ZUSAMMENSETZUNGEN MIT NIEDRIGDOSIERTEN WIRKSTOFFEN
COMPOSITIONS PHARMACEUTIQUES COMPRENANT DES MÉDICAMENTS FAIBLEMENT DOSÉS

(30) Priority: 06.02.2013 EP 13154303
(43) Date of publication of application: 16.12.2015
(73) Proprietor: Hermes Arzneimittel GmbH, 82049 Pullach (DE)
(72) Inventor: DANDL, Klaus, 82041 Oberhaching (DE); HAALA, Josef, 82049 Pullach (DE); HAACK, Detlev, 82049 Pullach (DE)
(74) Representative: Pharma Patents International AG
(86) International application number: PCT/EP2014/052286
(87) International publication number: WO 2014/122195

(56) References cited:
- JP-A- 2007 051 133
- JP-A- 2008 007 496
- US-A- 5 587 179

## Description

### FIELD OF THE INVENTION

The present invention relates to pharmaceutical compositions comprising active ingredients for oral use, and method for the manufacture of such compositions.

### BACKGROUND OF THE INVENTION

In pharmaceutical compositions for oral use, the incorporation of certain active ingredients may present the formulation development expert with substantial challenges, depending on the properties of the respective drug substance. For example, very poorly water-soluble compounds are difficult to incorporate in tablets and capsules in such a way that they dissolve fast enough to become completely absorbed in the upper gastrointestinal tract. Other challenging drug properties include poor chemical stability, hygroscopy, light sensitivity, sublimation, and the like. Moreover, it is difficult to formulate a drug whose single dose is above about 500 to 750 mg, in particular if that drug substance is at the same time poorly compressible. Together with the required excipients, the resulting dosage unit, e.g. tablet or capsule, will be so large that it may not be easily swallowable by the patient.

A further challenge is the incorporation of low-dose drugs into solid oral drug formulations. Since tablets and types other dosage forms must be safely and conveniently handled by the patient, they should have a certain minimum size, weight and strength. Even within a rather small tablet of only 50 to 100 mg of total weight, the fraction represented by the drug substance may be minute in the case of a low-dose drug whose single dose, e.g. 1 mg or less. It is a technological challenge to make powder or granule mixtures in which such small fraction is very evenly distributed. On the other hand, an even distribution is of great importance in order to achieve a sufficient content uniformity and dose accuracy.

Conventionally, these issues are tackled by first preparing granules from such low-dose active ingredients and appropriate fillers and binders at a moderate level of dilution. In a subsequent step, these granules are further diluted with other granules. The drawback of such two-step approach is that the preparation of the first granulation is associated with additional cost. Wet granulation requires subsequent drying, which involves substantial energy consumption. Dry granulation requires special equipment and is not always possible due to the physical properties of the raw materials.

Occasionally, it has been suggested to adsorb active ingredients onto inert excipient particles. Typically, these carrier particles are made of an inorganic material which is not water-soluble and which would not be suitable for certain types of formulations such as dispersible granules, dispersible tablets, effervescent tablets, and effervescent granules. For example, US 2006/182691 describes a composition comprising progesterone which is first dispersed in sunflower oil and then adsorbed to Sipernat© 50, which is a silica-based carrier.

Moreover, US 5,587,179 describes effervescent and dispersible granules and tablets in which the active ingredient is incorporated within a matrix including a fatty ester or a wax. The matrix contains cellulose derivatives such as hydroxypropylmethylcellulose phthalate, cellulose acetate phthalate, cellulose acetate trimellitate or cellulose acetobutyrate, and in particular at least one polymethacrylate, such as Eudragit L or Eudragit S, and is in mixture with common excipients such as starch or mannitol. The preparation of these granules relies on a multi-step process requiring dissolution in organic solvent and subsequent evaporation for embedding and uniformly distributing the active ingredient in the matrix and/or fatty ester or wax. The use of solid fatty esters or waxes, moreover, will lead to insoluble residues which easily separate from the aqueous phase after such formulation is placed in water.

There is a need for improved methods of incorporating low-dose drugs in pharmaceutical formulations for oral use which do not possess the disadvantages known in the arts. In particular, there is a need for simple, cost-effective methods for incorporating low-dose drugs in dispersible and effervescent dosage forms, and for the respective compositions.

It is an object of the invention to provide such improved compositions comprising a low-dose drug. Another object is to provide improved compositions comprising a drug which exhibits a small mean particle size. A further object is to provide improved compositions with a low-dose drug or a drug having a small particles size in the form of dispersible or effervescent tablets or granules. A yet further object is to provide methods for preparing such improved compositions. Other objects will become clear on the basis of the description and the claims.

### SUMMARY OF THE INVENTION

The invention provides a pharmaceutical powder or granule composition for oral use, comprising (a) an active ingredient at a level of not more than about 10 wt.-%; (b) a water-soluble, pharmaceutically acceptable carrier; and (c) an oily excipient which is liquid at room temperature; wherein the weight ratio of the water-soluble carrier to the oily excipient is at least about 5 to 1 and wherein the active ingredient adheres to the water-soluble carrier by means of the oily liquid.

The inventors have found that active ingredients which are usually difficult to incorporate homogeneously into powder or granule compositions because of their low dose or small particle size may be attached to carrier particles or granules by means of relatively small amounts of an oily liquid, even if the carrier is a water-soluble excipient. A mixture comprising the active ingredient attached to the carrier in this manner is substantially more homogeneous than a simple mixture obtained by dry mixing of the active ingredient and the carrier.

The composition may be used as such, i.e. as a powder or granule composition for oral use, or it may be further processed by the addition of further excipients. Optionally, such further excipients include an effervescent excipient or couple, and the final mixture may be compressed into an effervescent tablet. Without compression, such mixture may be used as effervescent granules presented e.g. in a sachet.

Suitable water-soluble carriers include, for example, monosaccharides, disaccharides, oligosaccharides, sugar alcohols, and monosodium citrate. Among the preferred disaccharides is sucrose, and among the preferred sugar alcohols are sorbitol and mannitol. Monosodium citrate is one of the preferred carriers when the final formulation is an effervescent dosage form.

Suitable oily excipients include triglyceride oils, such as medium-chain triglycerides, and other pharmaceutically acceptable oils such as simethicone.

The active ingredient may be attached to the carrier by either one of the following methods:
(1) Mixing the active ingredient and the water-soluble carrier to form a powder or granule mixture, followed by mixing the mixture obtained in the first step with the oily excipient.
(2) Mixing the water-soluble carrier and the oily excipient such as to obtain a powder or granule mixture, followed by mixing the mixture obtained in the first step with the active ingredient.
(3) Dispersing or dissolving the active ingredient in the oily excipient to form a liquid solution, dispersion or suspension, followed by mixing the water-soluble carrier with the liquid solution, dispersion or suspension obtained in the first step.

Further aspects and embodiments of the invention will become clear on the basis of the detailed description below, including the examples, and the patent claims.

### DETAILED DESCRIPTION OF THE INVENTION

In a first aspect, the invention provides a pharmaceutical powder or granule composition for oral use, comprising (a) an active ingredient at a level of not more than about 10 wt.-%; (b) a water-soluble, pharmaceutically acceptable carrier; and (c) an oily excipient which is liquid at room temperature; wherein the weight ratio of the water-soluble carrier to the oily excipient is at least about 5 to 1 and wherein the active ingredient adheres to the water-soluble carrier by means of the oily liquid.

As used herein, a pharmaceutical composition is a composition comprising at least one biologically active ingredient and at least one excipient that is, in the amount in that the excipient is incorporated in the composition, pharmacologically inert.

A powder is a composition in which a particulate material is dispersed in a gas phase, e.g. air. Typically, a powder exhibits some degree of flowability, e.g. characterised by a Hausner ratio of not more than 1.45, in particular of 1.4 or less, or even 1.35 or less, respectively. Granule compositions are a special form of a flowable powder in which at least some of the particles are in agglomerated form. As used herein, the generic term "powder" includes granules.

A composition for oral use is a pharmaceutical composition which is formulated and processed to be suitable for oral administration, or suitable to serve as a component or intermediate for a final product which is suitable for oral administration.

An active ingredient is a compound or mixture of compounds (as e.g. in the case of a plant extract) having a biological activity useful for the therapy, management, prevention or diagnosis of a disease, health condition or symptom. Synonyms for active ingredient include, without limitation, bioactive compound, drug substance, active pharmaceutical ingredient (API), active, pharmacological agent etc.

In the context of the invention, a carrier is understood as an excipient to which an active ingredient may be physically attached, such as by adsorption or adhesion, and which may also serve as a diluent or bulking agent.

As used herein, water-soluble means that a material dissolves in water or an aqueous liquid medium at room temperature. In particular, a water-soluble carrier has a solubility which is, according to the classification of the United States Pharmacopeia (USP), in the category "soluble" or better, i.e. "freely soluble" or "very soluble". According to this classification, "soluble" means that from 10 to 30 parts of solvent (here: water) are required to dissolve one part of the material; "freely soluble" means that from 1 to 10 parts of solvent are required for dissolution; and "very soluble" includes material for whose dissolution less than one part solvent is required per part of the material.

An oily excipient is a pharmaceutically acceptable material, comprising one or more compounds, which is substantially lipophilic and thus not freely miscible with water, even though some moderate degree of aqueous solubility may exist. The oily excipient is a liquid at room temperature.

The inventors have surprisingly found that active ingredients which are usually difficult to incorporate homogeneously into powder or granule compositions because of their low dose or small particle size may be attached to carrier particles or granules by means of relatively small amounts of an oily liquid, even if the carrier is a water-soluble excipient. A mixture comprising the active ingredient attached to the carrier in this manner is substantially more homogeneous than a simple mixture obtained by dry mixing of the active ingredient and the carrier. Thus, the invention provides a convenient and cost-effective means of achieving a homogeneous distribution of a low-dose active ingredient in a powder or granule formulation, or in a tablet such as an effervescent tablet, if the powder or granule composition is further processed into such compressed dosage form.

One of the advantages brought about by the invention is the improved quality of the resulting formulation in terms of content uniformity, ensuring the same reproducible amount of active ingredient in each dosage unit of the final formulation, e.g. in each individual tablet, sachet, or stick pack. Another advantage is that alternative processing methods with high energy consumption which might otherwise be required in order to ensure a sufficient homogeneity and content uniformity, such as dry or wet granulation with subsequent drying, roller compaction or the like, usually followed by grinding and/or sieving, may be avoided. A yet further advantage over prior art is that the composition of the invention is particularly suitable for dosage forms which are dissolved or dispersed in water prior to oral administration, such as effervescent tablets, effervescent granules, dispersible tablets or dispersible granules: Due to the water-soluble carrier, there is no or only very little insoluble residue (e.g. very small amounts of a tablet lubricant). In some embodiments, surprisingly, even the oily excipient is homogeneously dispersed in the water in very fine droplets rather that forming an oily film floating on the water.

The active ingredient, as mentioned, is preferably a low-dose drug and/or a material having a particularly small mean particle size. Both these properties make the incorporation into an oral pharmaceutical formulation very challenging in terms of processability and product quality. Both properties substantially increase the tendency and risk of de-mixing, leading to inhomogeneity and lack of content uniformity. A low-dose drug incorporated as a fine powder may also selectively adsorb to surfaces of processing equipment or even the primary packaging materials of the final dosage form.

As used herein, a low-dose drug is an active ingredient whose effective single dose is much smaller than the weight of an oral dosage unit suitable and convenient for handling and administration. For example, compressed tablets should normally have a diameter of at least about 5 mm and a height of at least about 2 mm in order to allow safe and convenient handling by the patient. These minimum dimensions roughly correspond to a tablet weight of at least about 40 mg. On the other hand, many drug substances have a single dose of less than 10 mg, or even 1 mg or less, which means that the formulation contains only a small fraction of active ingredient distributed in an excipient mixture. This problem is even more pronounced when the final formulation is an effervescent formulation, such as effervescent granules or an effervescent tablet, which would inherently require relatively large amounts of excipient, in particular of the effervescent couple, making the incorporation of a low-dose drug particularly challenging. A further level of difficulty results when a low-dose drug is incorporated in combination with a high-dose drug, e.g. a drug whose single dose is 200 mg or more, in particular if the final formulation is an effervescent tablet or effervescent granules.

Accordingly, in one of the preferred embodiments, the low-dose drug is present in the composition at an amount of 10 mg or less per dosage unit, or even at an amount of 5 mg or less, 2 mg or less, or even 1 mg or less, respectively, per dosage unit. In a further preferred embodiment, the composition represents, or is further processed into, an effervescent tablet or effervescent granules. In yet a further embodiment, the composition further comprises a high-dose drug at an amount of at least about 200 mg per dosage unit, or of at least about 250 mg, or at least about 300 mg, or at least about 400 mg, or at least about 500 mg, respectively, per dosage unit. The high-dose drug is preferably not attached to the water-soluble carrier through the oily excipient.

In any of these cases, the content of the (first) active ingredient in the composition is not more than about 10 wt.-%. In many cases, the content is substantially less, such as about 5 wt.-% or less, or not more than about 3 wt.-%, or not more than about 1 wt.-%, respectively. If the composition is used as a component of an effervescent formulation, such as an effervescent tablet or effervescent granules, the content of the active ingredient is preferably not more than about 5 wt.-% relative to the final formulation, or not more than about 3 wt.-%, or not more than about 1 wt.-%, or not more than about 0.5 wt.-%, respectively.

In a specific embodiment, the low-dose drug is defined by single dose of not more than about 10 mg or a content of not more than about 5 wt.-% in the dosage unit.

Similarly, a drug substance with a very low mean particle size is challenging to incorporate into a powder or granule mixture simply by dry mixing. Small particles tend to de-mix from larger particles such as granules, thus causing lack of content uniformity. If the small drug particles are also rather dense, the risk is even more pronounced. Due to their high surface energy, very fine powders show a higher propensity to adhere to other surfaces.

Accordingly, in one of the preferred embodiments, the active ingredient has a mean particles size of not more than about 80 µm. In further embodiments, the mean particle size is not more than about 50 µm, or not more than about 30 µm, or not more than about 20 µm, respectively.

The active ingredient may represent a single chemical entity, i.e. a chemically pure substance, or a mixture of compounds as typically present in e.g. herbal extracts. In one of the preferred embodiments, the active ingredient is a chemically pure substance.

With respect to the therapeutic indication, the active ingredient may in principle be any drug substance suitable for oral delivery. Among the preferred compounds are drugs useful as remedies for cough and cold, such as caffeine, chlorphenamine, phenylephrine, dextromethorphan, diphenhydramine, guaifenesin, ambroxol, dipropizine, pentoxyverine, and the like. For the avoidance of doubt, the compounds as mentioned herein should be understood so as to include any pharmaceutically acceptable salts thereof, such as chlorphenamine maleate or phenylephrine hydrochloride, only to mention some examples. One of the particularly preferred active ingredients according to the invention is phenylephrine hydrochloride at a dosage strength of about 0.5 to about 2.5 mg per dosage unit.

Other suitable drug substances include, without limitation, acarbose, cetirizine, levocetirizine, loperamide, dextromethorphan, diphenhydramine, dimenhydrinate, loratadine, desloratadine, memantine, adamantane, vitamin B compounds such as thiamine, riboflavin, niacin, pantothenic acid, vitamin B₆ (pyroxidine, pyridoxal and pyridoxamine), biotin, folic acid, vitamin B₁₂ (cobalamin); pseudoephedrine, ephedrine, phenylpropanolamine, ivy extract, frovatriptan, almotriptan, naratriptan, rizatriptan, eletriptan, zolmitriptan, including any pharmaceutically acceptable salts and derivatives thereof, as well as any mixtures thereof.

According to a specific embodiment, the active ingredient is a vitamin B₆ compound or mixture of compounds, such as pyroxidine or a mixture of pyroxidine with pyridoxal and/or pyridoxamine.

As mentioned, the key excipients according to the invention are a water-soluble carrier and an oily excipient which is liquid at room temperature. The water-soluble carrier may, for example, be a sugar, such as a monosaccharide, a disaccharide, or an oligosaccharide; or a sugar alcohol, or a soluble inorganic excipient such as monosodium citrate. Examples of suitable monosaccharides include glucose, fructose, galactose or xylose, among which glucose and fructose are preferred. Suitable disaccharides include sucrose, lactose, lactulose, maltose, trehalose, cellobiose and isomaltose, among which sucrose and lactose are preferred. Among the suitable sugar alcohols are sorbitol, mannitol, xylitol, lactitol, maltitol, erythritol, isomalt, and others, from which sorbitol and mannitol are currently preferred. Suitable inorganic carriers are in particular sodium salts of citric acid, tartaric acid, succinic acid, and malic acid, in particular monosodium citrate. Especially for the purpose of effervescent formulations, such acidic carrier is suitable in that it may simultaneously function as a component of the effervescent couple.

Advantageously, the water-soluble carrier particles have a relatively large specific surface area. It is believed that a large surface area corresponds to a relatively large capacity to adsorb the oily excipient, and this the active ingredient by means of the oily excipient. For example, the specific surface area may be about 1 m²/gram or more, or about 1.5 m²/gram or even about 2 m²/gram or more. In another embodiment, the mean particle size of the carrier is from about 200 µm to about 400 µm and the specific surface area is more than about 1.5 m²/gram. The inventors have surprisingly found that, in particular, this type of carrier material, when loaded with an active ingredient by means of an oily liquid excipient, is suitable for incorporation into an effervescent formulation. When placed into water, the effervescent formulation disintegrates and the oily liquid becomes very finely dispersed in the aqueous phase instead of coalescing on the surface of the water. The fine dispersion, or emulsion, may be stable over several hours.

A large surface area may be due to a small mean particle size and/or a high porosity of the carrier particles. Thus, the carrier particles may have a relatively large mean particle size, in particular relative to the active ingredient. For example, the mean particle size as determined by laser diffraction may be not only larger than that of the active ingredient, but also about 150 µm or more, such as about 200 µm or more, or in the range from about 100 µm to about 600 µm, in particular from about 150 µm to about 500 µm, such as from about 200 µm to about 400 µm. These particle sizes are often associated with good processability in terms of flowability and compressibility. At the same time, these particles may have a high surface area due to their porosity.

Optionally, the water-soluble carrier may represent an agglomerated or granulated material, such as a spray-dried or granulated sugar or sugar alcohol. In this case, the above mentioned mean particle sizes refer to the agglomerated particles or granules rather than the primary particles which may only be recovered by milling or the like.

An example for this type of water-soluble carrier is granulated sorbitol having a mean particle size in the range of approx. 250 to 300 µm and a specific surface area in the range of approx. 1.5 to 2.5 m²/g. Alternatively, other granulated sugars or sugar alcohols may be used instead of sorbitol.

Also suitable are water-soluble carriers with a large surface area due to a small mean particle size, as measure by laser diffraction, such as below about 120 µm, or below about 100 µm, such as in the range from about 20 µm to about 100 µm. Such carrier particles may or may not be porous. An example of such type of water-soluble carrier is monosodium citrate with a mean particle size in the range from about 50 µm to about 80 µm.

By weight, the amount of the water-soluble carrier is preferably at least about the same as the amount of the active ingredient. More preferably, the amount of the water-soluble carrier is larger than that of the active ingredient. For example, the weight ratio of the water-soluble carrier to the active ingredient may be at least about 2 to 1. In further preferred embodiments, this ratio is at least about 3 to 1, or at least about 5 to 1, respectively.

The oily excipient may be selected from lipophilic, pharmaceutically acceptable excipients that are liquid at room temperature and suitable for oral administration. As used herein, room temperature means normal or standard conditions with respect to temperature and pressure, also commonly referred to as ambient conditions. Preferably, room temperature should be interpreted as being in the range from 20 to 25 °C at a pressure of approximately 1,013 mbar (±40 mbar). In other words, the melting point (or the upper limit of the melting range) of the oily excipient is below 20 to 25 °C. Optionally, the melting point is not higher than about 15 °C at normal pressure.

As used herein, and as commonly understood, the melting point (or upper limit of the melting range) of an oily excipient refers to a value which is observed and determined, using melting-point measurement apparatus and tests known in the art, for the excipient as such. Thus the term melting point refers to a physical property of the excipient alone and not the physical property of the excipient observed when it is mixed together with other excipients or components. Where the excipient comprises more than one compound, the term melting point refers to a physical property of the excipient material as such (e.g. such as indicated in its product specification) and not the physical property of the excipient as a mixture with further or other compounds or excipients. For example, the triglyceride oil commonly sold as Miglyol® 810, which is a mixture of triglycerides of caprylic acid (65-80%) and capric acid (20-35%), has a melting point of approx. -15° C (with some variability due to differences in the content of fatty acid residues). Also preferred is an oily excipient which is liquid not only at room temperature, but also under refrigeration, such as at 2 to 8 °C, to allow easier handling and to avoid any crystallisation or phase changes during product storage which could create stability issues. In this case, the melting point (or the upper limit of the melting range) of the oily excipient is not higher than about 2 °C.

As mentioned above, oily means that the excipient is substantially lipophilic and thus not freely miscible with water, even though some moderate degree of aqueous solubility may exist. Lipophilic, as defined by IUPAC, literally means 'fat-loving' and refers to molecules having a tendency to dissolve in fat-like (e.g. hydrocarbon) solvents (IUPAC. Compendium of Chemical Terminology, 2nd ed. (the "Gold Book"). Compiled by A. D. McNaught and A. Wilkinson. Blackwell Scientific Publications, Oxford (1997). XML on-line corrected version: http://goldbook.iupac.org (2006-) created by M. Nic, J. Jirat, B. Kosata; updates compiled by A. Jenkins. ISBN 0-9678550-9-8. doi:10.1351/goldbook).

A commonly used measure of lipophilicity is Log P. Log P is the measurement of a compound's distribution between a non-aqueous (e.g. octanol) and aqueous (e.g. water) phase, and is also referred to as the partition coefficient or ratio. More specifically, it is the logarithm base 10 of the ratio of the concentration of a substance in a single definite form in the non-aqueous phase to its concentration in the same form in the aqueous phase at equilibrium. Where octanol and water are used as the respective phases, this measure is often referred to as the octanol-water partition coefficient; Log P_{ow}, or Log K_{ow}. The determination of Log P for a compound may be carried out by various measurement methods known in the art, for example by the shake-flask method or by HPLC. Log P, or Log P_{ow} may also be calculated using methods known in the art on the basis on the chemical structure of a compound. Preferably, the oily excipients have a log P of at least 4, or at least 6, or at least 8, respectively.

Moreover, oils are preferred which have a relatively low viscosity such as to enhance their spreading on the surface of the carrier particles when mixed together.

Viscosity is the measure of a fluid's resistance to flow stress. The dynamic viscosity of a fluid is a measure of its resistance to shear stress. Dynamic viscosity measurements may be determined at a given temperature using standard measurement techniques and apparatus known in the art for such purposes, for example, with viscometers and rheometers. Viscometers which may be used include glass capillary viscometers, falling ball viscometers, or rotational viscometers. In one embodiment of the invention, the preferred dynamic viscosity as determined at room temperature (20-25°C as defined above) is less than about 500 mPas. In another embodiment of the invention, the preferred dynamic viscosity at room temperature of the oily excipient is not more than about 55 mPas, not more than about 40 mPas, or not more than about 30 mPas. In a particularly preferred embodiment, the dynamic viscosity as determined at room temperature of the oily excipient is between about 15 mPas and about 30 mPas. In the event that the powder or granule composition is to be used in or for an effervescent formulation, such as an effervescent tablet or effervescent granules, it is one of the preferred options that the viscosity is selected to be not higher than that of mixed caprylic/capric triglycerides, e.g. Miglyol® 810 (approx. 26-32 mPas) or Miglyol® 812 (approx. 28-33 mPas).

The oily excipient should preferably be chemically stable and inert.

Examples of suitable oily excipients include liquid triglycerides, such as pure or mixed medium chain triglycerides, such as glyceryl tricaprylate (e.g. Miglyol® 808), mixed caprylic/capric triglycerides (e.g. Miglyol® 810 and 812), mixed caprylic/capric/succinic triglycerides (e.g. Miglyol® 829), propylene glycol dicaprylate/dicaprate (e.g. Miglyol® 840), as well as purified sesame oil, peanut oil, other natural oils, or any mixtures of these. The Miglyol® grades above have useful viscosities from about 10 to about 300 mPas, logP values in the range from about 6 to 10, and a melting point of less than 0 °C. From these, Miglyol® 808, Miglyol® 810, Miglyol® 812, and Miglyol® 829 are particularly preferred.

Alternatively, non-triglyceride oils may be used, provided that they are suitable for oral use in consideration of the amount incorporated into the composition of the invention, such as oleic acid, ethyl oleate, decyloleate, (light) mineral oil, dimethicone, or simethicone. In the case that unsaturated oils or fatty acids are used, such as oleic acid, it may be useful to also incorporate an antioxidant within the composition of the invention to minimise degradation of the oily excipient.

For the avoidance of doubt, the oily excipient may, depending on the amount in which it is incorporated, have some physiological activity, and may thus also be considered an active ingredient in some cases. For example, mineral oil is known to have a laxative effect, and simethicone is an anti-foaming agents used against bloating.

According to the invention, the amount of oily excipient is much lower than that of the water-soluble carrier. Preferably, the weight ratio between the two is at least about 5 to 1. Optionally, it may be at least 6 to 1, or at least 7 to 1, at least 8 to 1, or at least 10 to 1, respectively. According to another embodiment, the ratio is from about 5 to 1 to about 20 to 1. The maximum load capacity of the carrier for the oil differs for every combination of a specific carrier with a specific oily excipient, and is impacted not only by the chemical nature of the respective excipients, but also by the particles size, porosity and/or specific surface area of the carrier particles.

With respect to the weight ratio of the oily excipient to the active ingredient, this is preferably in the range from about 5 to 1 to about 1 to 2, and for example about 1 to 1, or about 2 to 1.

In certain embodiments, the oil which is attached to the water-soluble carrier particles will, upon contact with water, become finely and homogeneously dispersed in the aqueous phase. In the case of an effervescent formulation, this may lead to an opalescent appearance of the effervescent drinking solution. Typically, the fine dispersion is stable enough for the purpose of administration, i.e. the oil remains dispersed over a period of up to several hours. In other cases, the dispersion may be further stabilised by incorporating a surfactant into the composition. For example, the surfactant may be mixed with the liquid oily excipient prior to loading the water-soluble carrier with the oily excipient and the active ingredient. Suitable surfactants are those that are, in their required amounts, safe and suitable for oral administration, such as polysorbates (e.g. various grades of Tween®), polyethoxylated castor oil (e.g. Cremophor® EL, Kollophor® EL), poloxamers (e.g. various grades of Pluronic®), and the like.

The amount of surfactant is typically not higher than that of the oily excipient. In many cases, a much smaller amount will suffice, such as to yield a weight ratio of the oily excipient to the surfactant in the range between about 20 to 1 and about 2 to 1, or between about 15 to 1 and about 5 to 1.

Optionally, the composition of the invention may comprise one or more further excipients commonly used in oral drug formulations, such as one or more glidants, flow regulators, bulking agents, colouring agents, antioxidants, pH-modifiers, sweeteners, flavouring agents, wetting agents, and the like. If the composition is to be used in a final formulation which is an effervescent tablet or effervescent granules, an effervescent couple may be incorporated. As mentioned, the acidic member of the effervescent couple may also be used as the water-soluble carrier, e.g. monosodium citrate.

Generally speaking, an effervescent couple (or mixture) comprises at least one acidic agent and at least one carbon dioxide-releasing agent. Of course, more than one excipient of one type may also be present in an effervescent formulation. Acidic agents potentially useful in effervescent formulations include citric acid anhydrate, citric acid monohydrate, tartaric acid, malic acid, ascorbic acid, fumaric acid, monosodium phosphate, and monosodium citrate. Carbon dioxide-releasing agents include sodium carbonate, sodium bicarbonate, potassium bicarbonate, potassium carbonate, and calcium carbonate.

Moreover, the composition may also comprise at least one further active ingredient, or it may be further processed into a final formulation which comprises at least one further active ingredient. As mentioned above, the composition of the invention is particularly advantageous for incorporating a low-dose active ingredient into a large dosage unit, such as an effervescent tablet or effervescent granules, which also contain another active ingredient which is high-dose. For example, certain popular drug combinations used as cough and cold remedies include a low-dose drug and a high-dose drug. Examples are combinations of a high-dose analgesic or antipyretic drug such as aspirin or paracetamol with a low-dose antitussive or decongestant drug such as dextromethorphan or phenylephrine, or any salts thereof. Optionally, a further active ingredient such as caffeine or an anti-allergy agent may be present in the composition or the final formulation.

In a further aspect, the invention is directed to a pharmaceutical tablet compressed from a powder or granule mixture, wherein this mixture comprising the composition as described above. For example, an effervescent tablet may be compressed from a granulate which contains, as a component, a low-dose drug such as phenylephrine bound to a water-soluble carrier by means of an oil.

In yet a further aspect, the invention is directed to methods for preparing the composition as described above. In particular, three methods are provided.

According to a first method, the active ingredient and the water-soluble carrier are mixed such as to form a powder or granule mixture. In a subsequent step, the mixture obtained in the first step is mixed with the oily liquid.

The second method includes a first step comprising the mixing the water-soluble carrier and the oily excipient such as to obtain a powder or granule mixture, followed by a step of mixing the mixture obtained in the first step with the active ingredient.

In the third method, a first step is included in which the active ingredient is dispersed or dissolved in the oily excipient such as to form a liquid solution, dispersion or suspension. In a subsequent step, the liquid solution, dispersion or suspension obtained in the first step is mixed with the water-soluble carrier.

The mixing and blending step of the first and second method is preferably performed using equipment such as a free-fall mixer, Turbula® shaker-mixer, or horizontal mixer, or compulsory mixer, preferably without any compaction and/or milling, sieving and other particle-size reduction steps.

The resulting powder or granule mixture from the first step of these methods may also preferably be formed without the aid of solvents or addition of further additional liquid excipients.

The dispersion or dissolution of an active ingredient in an oily liquid excipient so as to form a liquid solution, dispersion or suspension may be performed using equipment that is suitable for the co-mixing of liquid and solid components.

In another aspect, the present invention is directed to a pharmaceutical powder or granule composition for oral use which is obtainable by the methods described above. In particular, the invention provides a pharmaceutical powder or granule composition for oral use, comprising (a) an active ingredient at a level of not more than about 10 wt.-%; (b) a water-soluble, pharmaceutically acceptable carrier; and (c) an oily excipient which is liquid at room temperature; wherein the weight ratio of the water-soluble carrier to the oily excipient is at least about 5 to 1, the composition being obtainable by a process comprising the steps of:
(i) mixing the active ingredient and the water-soluble carrier such as to form a powder or granule mixture, followed by
(ii) mixing the mixture obtained in step (i) with the oily liquid.

Moreover, the invention provides a pharmaceutical powder or granule composition for oral use, comprising (a) an active ingredient at a level of not more than about 10 wt.-%; (b) a water-soluble, pharmaceutically acceptable carrier; and (c) an oily excipient which is liquid at room temperature; wherein the weight ratio of the water-soluble carrier to the oily excipient is at least about 5 to 1, the composition being obtainable by a process comprising the steps of:
(i) mixing the water-soluble carrier and the oily excipient such as to obtain a powder or granule mixture, followed by
(ii) mixing the mixture obtained in step (i) with the active ingredient.

Furthermore, the invention provides a pharmaceutical powder or granule composition for oral use, comprising (a) an active ingredient at a level of not more than about 10 wt.-%; (b) a water-soluble, pharmaceutically acceptable carrier; and (c) an oily excipient which is liquid at room temperature; wherein the weight ratio of the water-soluble carrier to the oily excipient is at least about 5 to 1, the composition being obtainable by a process comprising the steps of:
(i) dispersing or dissolving the active ingredient in the oily excipient such as to form a liquid solution, dispersion or suspension, followed by
(ii) mixing the liquid solution, dispersion or suspension obtained in step (i) with the water-soluble carrier.

In this context, a liquid solution, dispersion or suspension means that the solution, dispersion or suspension is liquid at room temperature and under normal pressure, as described above.

With respect to further preferred features of the active ingredient, the water-soluble carrier, the liquid oily excipient, reference is made to the respective passages herein-above, as they also apply to this aspect of the invention.

Further embodiments, options, and/or preferences are illustrated by the following examples.

### EXAMPLES

### Example 1: Effervescent tablets with 500 mg paracetamol, 65 mg caffeine and 5 mg phenylephrine hydrochloride

### (a) Preparation of phenylephrine-HCl pre-mix

| | |
|---|---|
| Active ingredient: | Phenylephrine HCl |
| Water-soluble carrier: | Monosodium citrate (mean particle size -50-80 µm) |
| Oily excipient: | Miglyol® 808 |
| Batch size: | 14000 dose units |

| **mg/Dose** | **Component** | **Total weight (g)** |
|---|---|---|
| 45.00 | Monosodium citrate | 630.000 |
| 5.00 | Miglyol 808 | 70.000 |
| 5.00 | Phenylephrine HCl | 70.000 |
| 55.00 | Phenylephrine HCl pre-mix | 770.000 |

Finely powdered monosodium citrate (e.g. with an average particle size of about 0.05 mm, such as about 0.05 to 0.08 mm) was mixed with finely powdered phenylephrine HCl (e.g. with an average particle size of about 0.03 mm) in a compulsory mixer for 5 min. Miglyol® 808 was then added, with further 5 min of mixing. The resulting mixture was then used in the preparation of the effervescent granulate.

### (b) Preparation of an effervescent granulate

**Batch size: 3800 dose units**

| **mg /Dose** | **Component** | **Total weight (g)** |
|---|---|---|
| 55.00 | Phenylephrine HCl pre-mix | 209.00 |
| 65.00 | Caffeine | 247.00 |
| 3094.37 | Effervescent base with paracetamol | 11758.61 |
| 62.90 | Flavouring and sweeteners | 239.02 |
| 3277.27 | Effervescent granulate | 12453.63 |

The effervescent granulate was compressed with a suitable tablet press to give effervescent tablets with a diameter of 25 mm. The tablets were then packaged into tablet tubes or blister packs.

### (c) Analytical results

**Drug content:**

| | |
|---|---|
| Expected: | 5.0 mg phenylephrine-HCl |
| Found: | 5.0 mg phenylephrine-HCl |

**Distribution in the effervescent tablets:**

| | |
|---|---|
| Variation coefficient: | CV = 1.03% (n=10) |
| Acceptance value (ref. Ph. Eur. 2.9.40): | 2.5 (conditions fulfilled) |

Comparative example: Effervescent tablets with 500 mg paracetamol, 65 mg caffeine and 5 mg phenylephrine hydrochloride without an oily excipient

For comparison purposes, a similar effervescent granulate and tablet as in the previous Example 1 was prepared in order to examine the effect of the oily excipient on the distribution of the active ingredient in the granulate formulation. The same process as described in Example 1 was performed with the exception of addition of the oily excipient, Miglyol® 808, which was replaced with the equivalent amount of sorbitol in the preparation of the final effervescent granulate mixture.

### (a) Preparation of a phenylephrine-HCl pre-mix

| | |
|---|---|
| Carrier: | Monosodium citrate (mean particle size about 50-80 µm) |
| Active ingredient: | Phenylephrine-HCl |
| Batch size: | 14000 dose units |

| **mg /dose** | **Component** | **Total Weight (g)** |
|---|---|---|
| 45.00 | Monosodium citrate | 630.000 |
| 5.00 | Phenylephrine HCl | 70.000 |
| 50.00 | Phenylephrine HCl pre-mix | 700.000 |

Finely powdered monosodium citrate (e.g. with an average particle size of 0.05 mm) was mixed with finely powdered phenylephrine HCl (e.g. with an average particle size of 0.03 mm) in a compulsory mixer for 5 min. The resulting mixture was used in the preparation of the effervescent granulate.

### (b) Preparation of effervescent granulate

**Batch size: 3750 dose units**

| **mg /dose** | **Component** | **Total weight (g)** |
|---|---|---|
| 50.00 | Phenylephrine HCl pre-mix | 187.500 |
| 65.00 | Caffeine | 247.000 |
| 3099.37 | Effervescent base with paracetamol | 11622.638 |
| 62.90 | Flavouring and sweeteners | 235.875 |
| 3277.27 | Effervescent granulate | 12289.763 |

The effervescent granulate was compressed with a suitable tablet press to give effervescent tablets with a diameter of 25 mm. The tablets were then packaged into tablet tubes or blister packs.

### (c) Analytical results:

**Drug content:**

| | |
|---|---|
| Expected: | 5.0 mg phenylephrine-HCl |
| Found: | 5.0 mg phenylephrine-HCl |

**Drug distribution in the effervescent tablets:**

| | |
|---|---|
| Variation coefficient: | CV = 6.99% (n=10) |
| Acceptance value (ref. Ph. Eur. 2.9.40): | 16.9 (conditions not fulfilled) |

### Example 2: Orally disintegrating histidine granulate

### (a) Preparation of histidine pre-mix

| | |
|---|---|
| Carrier: | Sorbitol, granulated (mean particle size approx. 280 µm, specific surface area approx. 2.1 m²/g) |
| Active ingredient: | Histidine |
| Oily excipient: | Miglyol® 812 |
| Batch size: | 2000 dose units |

| **mg /dose** | **Component** | **Total Weight (kg)** |
|---|---|---|
| 750.00 | Sorbitol, granulated | 1.500 |
| 5.00 | Miglyol 812 | 0.010 |
| 50.00 | Histidine powder | 0.110 |
| 805.00 | Histidine pre-mix | 1.620 |

Sorbitol was mixed with Miglyol® 812 in free-fall mixer for 10 min. Histidine powder was then added and mixing was continued for a further 10 min. The resulting mixture was used for preparation of the orally disintegrating granulate.

### (b) Preparation of histidine granulate

| **mg /dose** | **Component** | **Total Weight (kg)** |
|---|---|---|
| 805.00 | Histidine pre-mix | 1.610 |
| 395.00 | Granulate mixture | 0.790 |
| 1200.00 | Histidine granulate | 2.400 |

### (c) Analytical results:

**Drug content:**

| | |
|---|---|
| Expected: | 50.0 mg histidine |
| Found: | 52.0 mg histidine, variation coefficient CV = 4.25% (n=10) |

In comparison, the distribution of histidine in similarly prepared mixture without the oily excipient was found to be substantially less homogeneous:

**Drug content (without Miglyol® 812):**

| | |
|---|---|
| Expected: | 50.0 mg histidine |
| Found: | 51.2 mg histidine, variation coefficient CV = 14.98% (n=10) |

### Example 3: Orally disintegrating ginseng and guarana extract granulate

| | |
|---|---|
| Carrier: | Sorbitol, granulated (mean particle size approx. 280 µm, specific surface area approx. 2.1 m²/g) |
| Active ingredient 1: | Ginseng extract powder |
| Active ingredient 2: | Guarana extract powder |
| Oily excipient: | Miglyol® 812 |
| Batch size: | Ginseng-guarana extract pre-mix 755000 dose units; final granulate 690000 dose units |

### (a) Preparation of ginseng-guarana pre-mix

| **mg /dose** | **Component** | **Total Weight (kg)** |
|---|---|---|
| 300.00 | Sorbitol, granulated | 226.500 |
| 30.00 | Miglyol 812 | 22.65 |
| 3.00 | Silicon dioxide, highly dispersed | 2.265 |
| 37.50 | Guarana extract powder | 37.75 |
| 50.00 | Ginseng extract powder | 28.313 |
| 420.50 | Ginseng-Guarana pre-mix | 317.478 |

Sorbitol was mixed with Miglyol® 812 in a horizontal mixer for 6 min. To this mixture was then added a pre-mixed combination of the guarana extract and ginseng extract powders and highly dispersed silicon dioxide. Mixing was continued for 10 min. The resulting mixture was used in the preparation of the orally disintegrating granulate.

### (b) Preparation of ginseng-guarana granulate

| **mg /dose** | **Component** | **Total Weight (kg)** |
|---|---|---|
| 420.50 | Ginseng-Guarana pre-mix | 290.145 |
| 1079.50 | Granulate mixture | 744.855 |
| 1500.00 | Ginseng-Guarana granulate | 1035.000 |

### (c) Analytical results:

**Drug content:**

| | |
|---|---|
| Expected: | 50.0 mg ginseng extract |
| Found: | 50.1 mg ginseng extract, variation coefficient CV = 0.64% (n=3) |

In comparison, distribution of the extract powders in similarly prepared mixture (without the oily excipient) was substantially less homogeneous. The uneven distribution of the brown-coloured extract powders could be readily observed by visual inspection of the final granulate with no oily excipient.

### Example 4: Phenylephrine HCl chewing gum tablets

### (a) Preparation of a phenylephrine-HCl pre-mix

| | |
|---|---|
| Carrier: | Sucrose, granulated |
| Active ingredient: | Phenylephrine-HCl |
| Oily excipient: | Miglyol® 812 |
| Batch size: | 500 dose units |

| **mg /dose** | **Component** | **Total Weight (g)** |
|---|---|---|
| 95.00 | Sucrose, granulated | 47.500 |
| 3.00 | Miglyol 812 | 1.500 |
| 2.00 | Phenylephrine HCl | 1.000 |
| 100.00 | Phenylephrine HCl pre-mix | 50.000 |

The sucrose was mixed with phenylephrine HCl for 5 min. The oily excipient Miglyol® 812 was then added and mixing was continued for a further 5 min. The resulting mixture was used for preparation of the chewing gum composition.

### (b) Preparation of chewing gum composition

| **mg /dose** | **Component** | **Total Weight (g)** |
|---|---|---|
| 1340.00 | Chewing gum base | 670.00 |
| 100.00 | Phenylephrine HCl pre-mix | 50.00 |
| 1440.00 | Chewing gum tablet compression material | 720.00 |

### (c) Analytical results:

**Drug content:**

| | |
|---|---|
| Expected: | 100 mg phenylephrine HCl |
| Found: | 102 mg phenylephrine HCl, variation coefficient CV = 4.5% (n=10) |

In comparison, the distribution of phenylephrine HCl in a similarly prepared mixture without the oily excipient was found to be substantially inhomogeneous:

**Drug content:**

| | |
|---|---|
| Expected: | 100 mg phenylephrine HCl |
| Found: | 155 mg phenylephrine HCl, variation coefficient CV = 25.8% (n=10) |

### Example 5: Orally disintegrating acarbose granulate

### (a) Preparation of acarbose pre-mix (process A)

| | |
|---|---|
| Carrier: | Sorbitol, granulated (mean particle size approx. 280 µm, specific surface area approx. 2.1 m²/g) |
| Active ingredient: | Acarbose |
| Oily excipient: | Simethicone |
| Batch size: | Acarbose pre-mix 150000 dose units; Acarbose granulate 145000 dose units |

| **mg /dose** | **Component** | **Total Weight (kg)** |
|---|---|---|
| 648.50 | Sorbitol, granulated | 97.275 |
| 125.00 | Simethicone | 18.938 |
| 50.00 | Acarbose | 7.500 |
| 823.50 | Acarbose pre-mix | 123.713 |

Sorbitol was mixed with acarbose for 15 min. The oily excipient simethicone was then added and mixing was continued for a further 20 min. The resulting mixture was used for preparation of the orally disintegrating granulate.

### (b) Preparation of acarbose granulate

| **mg /dose** | **Component** | **Total Weight (g)** |
|---|---|---|
| 26.50 | Granulate mixture | 3.842 |
| 823.50 | Acarbose pre-mix | 119.408 |
| 850.00 | Acarbose granulate | 123.250 |

### (c) Analytical results:

**Drug content:**

| | |
|---|---|
| Expected: | 50.0 mg acarbose |
| Found: | 50.0 mg acarbose, variation coefficient CV = 1.34% (n=10) |

### (d) Preparation of acarbose pre-mix (process B)

| | |
|---|---|
| Carrier: | Sorbitol, granulated (mean particle size approx. 280 µm, specific surface area approx. 2.1 m²/g) |
| Active ingredient: | Acarbose |
| Oily excipient: | Simethicone |
| Batch size: | 200 dose units |

| **mg /dose** | **Component** |
|---|---|
| 648.50 | Sorbitol, granulated |
| 125.00 | Simethicone |
| 50.00 | Acarbose |
| 823.50 | Acarbose pre-mix |

Acarbose was suspended in simethicone under vigorous stirring. The resulting suspension was added to sorbitol and the resulting mixture was mixed for 10 min.

### (e) Analytical results:

**Drug content:**

| | |
|---|---|
| Expected: | 50.0 mg acarbose |
| Found: | 51.5 mg acarbose, with a variation coefficient of CV = 0.56% (n=6) |

### Example 6: Cranberry extract granulate

| **mg /dose** | **Component** | **Total weight (g)** |
|---|---|---|
| 300.00 | Cranberry juice concentrate powder | 15.000 |
| 100.00 | Pomegranate juice powder | 5.000 |
| 656.00 | Sorbitol Neosorb P300 DC | 32.800 |
| 20.00 | Miglyol 812 | 1.000 |
| 1076.00 | Cranberry extract granulate | 53.800 |

Sorbitol was mixed with Miglyol® 812 for 15 min in a Turbula shaker-mixer. The two extract powders were then added and the resulting mixture was further mixed for 10 minutes to afford a granulate with homogenously distributed juice extracts. In comparison, the mixing of all of the above components with the exception of oily excipient Miglyol® 812 resulted in a granulate in which the finely powdered extract components was poorly distributed. The substantial difference in homogeneity between the two preparations was apparent at visual inspection.

### Example 7: Effervescent tablets with 500 mg Vitamin C and 25 mg Dextromethorphan-HBR

### (a) Preparation of a Dextromethorphan-HBR pre-mix

| | |
|---|---|
| Active ingredient: | Dextromethorphan-HBR |
| Water-soluble carrier: | Sorbitol |
| Oily excipient: | Miglyol® 808 |
| Batch size: | 4900 dose units |

| **mg /dose** | **Component** | **Total Weight (g)** |
|---|---|---|
| 50.00 | Sorbitol | 245.00 |
| 5.75 | Miglyol® 808 | 28.18 |
| 25.00 | Dextromethorphan-HBR | 122.50 |
| 80.75 | Dextromethorphan-HBr oily phase | 395.68 |

Sorbitol (e.g. with an average particle size of 0.3 mm) was mixed with finely powdered dextromethorphan-HBr (e.g. with an average particle size of 0.03 mm) in a rotary blender for 5 min. Miglyol® 808 was then added, with further 5 min of mixing. The resulting mixture was then used in the preparation of the effervescent granulate.

### (b) Preparation of an effervescent granulate

**Batch size: 4900 dose units**

| **mg /dose** | **Component** | **Total Weight (g)** |
|---|---|---|
| 80.75 | Dextromethorphan-HBr oily phase | 395.68 |
| 1851.25 | Effervescent base with 500 mg Vitamin C | 9017.13 |
| 68.00 | Flavouring and sweeteners | 333.20 |
| 2000.00 | Effervescent granulate | 9800.01 |

The effervescent granulate was compressed with a suitable tablet press to give effervescent tablets with a diameter of 20 mm. The tablets were then packaged into tablet tubes or blister packs.

### (c) Analytical results:

Distribution of dextromethorphan-HBr in the effervescent tablets:
Variation coefficient: CV = 2.52% (n=10)

### Comparative example: Effervescent tablets with 500 mg Vitamin C and 25 mg Dextromethorphan-HBr without an oily excipient

For comparison purposes, a similar effervescent granulate and tablet as in the previous Example 7 was prepared in order to examine the effect of the oily excipient on the distribution of the active ingredient in the granulate formulation. The same process as described in Example 7 was performed with the exception of addition of the oily excipient, Miglyol® 808, which was replaced with the equivalent amount of sorbitol in the preparation of the final effervescent granulate mixture.

### (a) Preparation of a Dextromethorphan-HBr pre-mix

| | |
|---|---|
| Active ingredient: | Dextromethorphan-HBr |
| Carrier: | Sorbitol |
| Batch size: | 4000 dose units |

| **mg /dose** | **Component** | **Total Weight (g)** |
|---|---|---|
| 50.00 | Sorbitol | 200.00 |
| 25.00 | Dextromethorphan-HBr | 100.00 |
| 75.00 | Dextromethorphan-HBr pre-mix | 300.00 |

Sorbitol (e.g. with an average particle size of 0.3 mm) was mixed with finely powdered dextromethorphan-HBr (e.g. with an average particle size of 0.03 mm) in a rotary blender for 5 min. The resulting mixture was then used in the preparation of the effervescent granulate.

### (b) Preparation of an effervescent granulate

**Batch size: 4000 dose units**

| **mg /dose** | **Component** | **Total Weight (g)** |
|---|---|---|
| 75.00 | Dextromethorphan-HBr pre-mix | 300.00 |
| 1857.00 | Effervescent base with 500 mg Vitamin C | 7428.00 |
| 68.00 | Flavouring and sweeteners | 272.00 |
| 2000.00 | Effervescent granulate | 8000.00 |

The effervescent granulate was compressed with a suitable tablet press to give effervescent tablets with a diameter of 20 mm. The tablets were then packaged into tablet tubes or blister packs.

### (c) Analytical results:

Distribution of dextromethorphan-HBr in the effervescent tablets:
Variation coefficient: CV = 4.58% (n=10)

### Example 8: Effervescent tablets with 450 mg calcium and 10 mg cetirizine dihydrochloride

### (a) Preparation of a cetirizine pre-mix

| | |
|---|---|
| Active ingredient: | Cetirizine dihydrochloride |
| Water-soluble carrier: | Monosodium citrate |
| Oily excipient: | Miglyol® 808 |
| Batch size: | 6600 dose units |

| **mg /dose** | **Component** | **Total Weight (g)** |
|---|---|---|
| 90.00 | Monosodium citrate | 594.00 |
| 10.00 | Miglyol® 808 | 66.00 |
| 10.00 | Cetirizine dihydrochloride | 66.00 |
| 110.00 | Cetirizine dihydrochloride oily phase | 726.00 |

Finely powdered monosodium citrate (e.g. with an average particle size of 0.05 mm) was mixed with finely powdered cetirizine dihydrochloride (e.g. with an average particle size of 0.03 mm) in a compulsory mixer for 5 min. Miglyol® 808 was then added, with a further 6 min of mixing. The resulting mixture was then used in the preparation of an effervescent granulate.

### (b) Preparation of an effervescent granulate

**Batch size: 1900 dose units**

| **mg /dose** | **Component** | **Total Weight (g)** |
|---|---|---|
| 110.00 | Cetirizine dihydrochloride oily phase | 209.00 |
| 80.00 | Fumaric acid | 152.00 |
| 3282.00 | Effervescent base with 450 mg calcium | 6235.80 |
| 338.0 | Mannitol | 642.20 |
| 40.00 | Flavouring | 76.00 |
| 3850.00 | Effervescent granulate | 7315.00 |

The effervescent granulate was compressed with a suitable tablet press to give effervescent tablets with a diameter of 25 mm. The tablets were then packaged into tablet tubes or blister packs.

### (c) Analytical results:

Distribution in the effervescent tablets:
Variation coefficient: CV = 2.73% (n=10)

### Comparative example: Effervescent tablets with 450 mg Calcium and 10 mg cetirizine without an oily excipient

For comparison purposes, a similar effervescent granulate and tablet as in the previous Example 8 was prepared in order to examine the effect of the oily excipient on the distribution of the active ingredient in the granulate formulation. The same process as described in Example 8 was performed with the exception of addition of the oily excipient, Miglyol® 808, which was replaced with an equivalent amount of mannitol in the preparation of the final effervescent granulate mixture.

### (a) Preparation of a cetirizine dihydrochloride pre-mix

| | |
|---|---|
| Active ingredient: | Cetirizine dihydrochloride |
| Carrier: | Monosodium citrate |
| Batch size: | 1900 dose units |

| **mg /dose** | **Component** | **Total Weight (g)** |
|---|---|---|
| 90.00 | Monosodium citrate | 171.00 |
| 10.00 | Cetirizine dihydrochloride | 19.00 |
| 100.00 | Cetirizine dihydrochloride pre-mix | 190.00 |

Finely powdered monosodium citrate (e.g. with an average particle size of 0.05 mm) was mixed with finely powdered cetirizine dihydrochloride (e.g. with an average particle size of 0.03 mm) in a compulsory mixer for 5 min. The resulting mixture was then used in the preparation of an effervescent granulate.

### (b) Preparation of effervescent granulate

**Batch size: 1900 dose units**

| **mg /dose** | **Component** | **Total Weight (g)** |
|---|---|---|
| 100.00 | Cetirizine dihydrochloride pre-mix | 190.00 |
| 80.00 | Fumaric acid | 152.00 |
| 3282.00 | Effervescent base with 450 mg calcium | 6235.80 |
| 348.00 | Mannitol | 661.20 |
| 40.00 | Flavouring | 76.00 |
| 3277.27 | Effervescent granulate | 7315.00 |

The effervescent granulate was compressed with a suitable tablet press to give effervescent tablets with a diameter of 25 mm. The tablets were then packaged into tablet tubes or blister packs.

### (c) Analytical results:

Distribution in the effervescent tablets:
Variation coefficient: CV = 4.03% (n=10)

### Example 9: Orally disintegrating vitamin B₆ and magnesium granulate

### (a) Preparation of vitamin B₆ pre-mix

| | |
|---|---|
| Carrier: | Maltitol, granulated |
| Active ingredient: | Pyridoxine HCl (vitamin B₆) |
| Oily excipient: | Miglyol® 812 |
| Batch size: | 20000 dose units |

| **mg /dose** | **Component** | **Total Weight (g)** |
|---|---|---|
| 20.350 | Maltitol, granulated | 407.000 |
| 1.000 | Miglyol 812 | 20.000 |
| 0.854 | Pyridoxine HCl (0.7 mg Vit. B₆) | 17.080 |
| 22.204 | Vitamin B₆ premix | 444.080 |

Maltitol was mixed with Miglyol® 812 in free-fall mixer for 15 min. Pyridoxine HCl powder was then added and mixing was continued for a further 10 min. The resulting mixture was used for preparation of the orally disintegrating granulate.

### (b) Preparation of vitamin B₆ and magnesium granulate

| **mg /dose** | **Component** | **Total Weight (kg)** |
|---|---|---|
| 22.204 | Vitamin B₆ premix | 13.322 |
| 1677.796 | Magnesium Granulate mixture | 1006.678 |
| 1700.000 | Vitamin B₆ and Magnesium granulate | 1020.000 |

### (c) Analytical results:

**Drug content:**

| | |
|---|---|
| Expected: | 0.7 mg vitamin B₆ |
| Found: | 0.70 mg vitamin B₆, with a variation coefficient of CV = 5.10% (n=10) |

In comparison, the distribution of vitamin B₆ in a similarly prepared mixture without the oily excipient was found to be substantially less homogeneous:

**Drug content (without Miglyol® 812):**

| | |
|---|---|
| Expected: | 0.7 mg vitamin B₆ |
| Found: | 0.64 mg vitamin B₆, with a variation coefficient of CV = 9.92% (n=10) |

## Claims

1. A pharmaceutical powder or granule composition for oral use, comprising
(a) an active ingredient at a level of not more than 10 wt.-%;
(b) a water-soluble, pharmaceutically acceptable carrier; and
(c) an oily excipient which is liquid at room temperature; wherein
the weight ratio of the water-soluble carrier to the oily excipient is at least 5 to 1 and wherein the active ingredient adheres to the water-soluble carrier by means of the oily liquid.

2. The composition of claim 1, wherein the weight ratio of the water-soluble carrier to the active ingredient is at least 2 to 1.

3. The composition of claim 1 or 2, wherein the mean particle size of the active ingredient is smaller than the mean particle size of the water-soluble carrier.

4. The composition of any preceding claim, wherein the active ingredient is a single chemical entity.

5. The composition of any preceding claim, wherein the water-soluble carrier is selected from monosaccharides, disaccharides, oligosaccharides, sugar alcohols, and monosodium citrate.

6. The composition of any preceding claim, wherein the water-soluble carrier has a mean particle size of at least 150 µm, and/or a specific surface area of at least 1 m² per gram.

7. The composition of any preceding claim, wherein the oily liquid is selected from triglyceride oils and simethicone.

8. The composition of any preceding claim, wherein the active ingredient is selected from, phenylephrine, vitamin B₆, acarbose, and plant extracts.

9. The composition of any preceding claim, wherein the active ingredient is phenylephrine hydrochloride, the carrier is monosodium citrate, and the oily liquid is medium-chain triglycerides.

10. The composition of any preceding claim, further comprising an effervescent excipient or couple.

11. The composition of any preceding claim, comprising a further active ingredient at an amount corresponding to at least 200 mg per dose unit.

12. A pharmaceutical tablet compressed from a powder or granule mixture, said mixture comprising the composition of any preceding claim.

13. A method for the preparation of the composition of claims 1 to 11, comprising the steps of
(a) mixing the active ingredient and the water-soluble carrier to form a powder or granule mixture, followed by
(b) mixing the mixture obtained in step (a) with the oily liquid.

14. A method for the preparation of the composition of claims 1 to 11, comprising the steps of
(a) dispersing or dissolving the active ingredient in the oily liquid to form a liquid solution, dispersion or suspension, followed by
(b) mixing the water-soluble carrier with the liquid solution, dispersion or suspension obtained in step (a).

15. A method for the preparation of the composition of claims 1 to 11, comprising the steps of
(a) mixing the water-soluble carrier and the oily liquid such as to obtain a powder or granule mixture; followed by
(b) mixing the mixture obtained in step (a) with the active ingredient.

16. The method according to any one of claims 13 to 15, wherein step (a) is performed without the aid of solvents or addition of further liquid excipients.

## Patentansprüche

1. Pharmazeutische Pulver- oder Granulatzusammensetzung zur oralen Anwendung, umfassend
(a) einen Wirkstoff in einer Konzentration von nicht mehr als 10 Gew.-%;
(b) einen wasserlöslichen pharmazeutisch unbedenklichen Träger; und
(c) einen öligen Hilfsstoff, der bei Raumtemperatur flüssig ist;
wobei das Gewichtsverhältnis von wasserlöslichem Träger zum öligen Hilfsstoff mindestens 5:1 beträgt und wobei der Wirkstoff mittels der öligen Flüssigkeit am wasserlöslichen Träger anhaftet.

2. Zusammensetzung nach Anspruch 1, wobei das Gewichtsverhältnis von wasserlöslichem Träger zum Wirkstoff mindestens 2:1 beträgt.

3. Zusammensetzung nach Anspruch 1 oder 2, wobei die mittlere Teilchengröße des Wirkstoffs kleiner ist als die mittlere Teilchengröße des wasserlöslichen Trägers.

4. Zusammensetzung nach einem der vorhergehenden Ansprüche, wobei es sich bei dem Wirkstoff um eine einzelne chemische Einheit handelt.

5. Zusammensetzung nach einem der vorhergehenden Ansprüche, wobei der wasserlösliche Träger aus Monosacchariden, Disacchariden, Oligosacchariden, Zuckeralkoholen und Mononatriumcitrat ausgewählt ist.

6. Zusammensetzung nach einem der vorhergehenden Ansprüche, wobei der wasserlösliche Träger eine mittlere Teilchengröße von mindestens 150 µm und/oder eine spezifische Oberfläche von mindestens 1 m² pro Gramm aufweist.

7. Zusammensetzung nach einem der vorhergehenden Ansprüche, wobei die ölige Flüssigkeit aus Triglycerid-Ölen und Simethicon ausgewählt ist.

8. Zusammensetzung nach einem der vorhergehenden Ansprüche, wobei der Wirkstoff aus Phenylephrin, Vitamin B₆, Acarbose und Pflanzenextrakten ausgewählt ist.

9. Zusammensetzung nach einem der vorhergehenden Ansprüche, wobei es sich bei dem Wirkstoff um Phenylephrinhydrochlorid, bei dem Träger um Mononatriumcitrat und bei der öligen Flüssigkeit um mittelkettige Triglyceride handelt.

10. Zusammensetzung nach einem der vorhergehenden Ansprüche, weiterhin umfassend einen Brausehilfsstoff oder ein Brausepaar.

11. Zusammensetzung nach einem der vorhergehenden Ansprüche, umfassend einen weiteren Wirkstoff in einer Menge, die mindestens 200 mg pro Dosiseinheit entspricht.

12. Pharmazeutische Tablette, gepresst aus einer Pulver- oder Granulatmischung, wobei die Mischung die Zusammensetzung nach einem der vorhergehenden Ansprüche umfasst.

13. Verfahren zur Herstellung der Zusammensetzung nach Ansprüchen 1 bis 11, welches die folgenden Schritte umfasst:
(a) das Mischen von Wirkstoff und wasserlöslichem Träger unter Bildung einer Pulver- oder Granulatmischung; gefolgt von
(b) dem Mischen der in Schritt (a) erhaltenen Mischung mit der öligen Flüssigkeit.

14. Verfahren zur Herstellung der Zusammensetzung nach Ansprüchen 1 bis 11, welches die folgenden Schritte umfasst:
(a) das Dispergieren oder Lösen des Wirkstoffs in der öligen Flüssigkeit unter Bildung einer flüssigen Lösung, Dispersion oder Suspension; gefolgt von
(b) dem Mischen des wasserlöslichen Trägers mit der in Schritt (a) erhaltenen flüssigen Lösung, Dispersion bzw. Suspension.

15. Verfahren zur Herstellung der Zusammensetzung nach Ansprüchen 1 bis 11, welches die folgenden Schritte umfasst:
(a) das Mischen des wasserlöslichen Trägers und der öligen Flüssigkeit, so dass man eine Pulver- oder Granulatmischung erhält; gefolgt von
(b) dem Mischen der in Schritt (a) erhaltenen Mischung mit dem Wirkstoff.

16. Verfahren nach einem der Ansprüche 13 bis 15, wobei Schritt (a) ohne Hilfe von Lösungsmitteln oder Zugabe weiterer flüssiger Hilfsstoffe durchgeführt wird.

## Revendications

1. Composition pharmaceutique en poudre ou en granulés à usage oral, comprenant
(a) un principe actif dont la teneur ne dépasse pas 10 % en poids ;
(b) un support hydrosoluble pharmaceutiquement acceptable ; et
(c) un excipient huileux qui est liquide à la température ambiante ;
le rapport en poids du support hydrosoluble à l'excipient huileux étant d'au moins 5 à 1 et le principe actif adhérant au support hydrosoluble au moyen du liquide huileux.

2. Composition selon la revendication 1, le rapport en poids du support hydrosoluble au principe actif étant d'au moins 2 à 1.

3. Composition selon la revendication 1 ou 2, la taille de particules moyenne du principe actif étant inférieure à la taille de particules moyenne du support hydrosoluble.

4. Composition selon l'une quelconque des revendications précédentes, le principe actif étant une entité chimique unique.

5. Composition selon l'une quelconque des revendications précédentes, le support hydrosoluble étant choisi parmi les monosaccharides, les disaccharides, les oligosaccharides, les alcools de sucre et le citrate monosodique.

6. Composition selon l'une quelconque des revendications précédentes, le support hydrosoluble ayant une taille de particules moyenne d'au moins 150 µm et/ou une surface spécifique d'au moins 1 m² par gramme.

7. Composition selon l'une quelconque des revendications précédentes, le liquide huileux étant choisi parmi les huiles de triglycérides et la siméthicone.

8. Composition selon l'une quelconque des revendications précédentes, le principe actif étant choisi parmi la phényléphrine, la vitamine B6, l'acarbose et des extraits de plantes.

9. Composition selon l'une quelconque des revendications précédentes, le principe actif étant le chlorhydrate de phényléphrine, le support étant le citrate monosodique et le liquide huileux étant des triglycérides à chaîne moyenne.

10. Composition selon l'une quelconque des revendications précédentes, comprenant en outre un excipient ou un couple effervescent.

11. Composition selon l'une quelconque des revendications précédentes, comprenant un autre principe actif dans une quantité correspondant à au moins 200 mg par unité de dose.

12. Tablette pharmaceutique comprimée à partir d'un mélange de poudres ou de granulés, ledit mélange comprenant la composition selon l'une quelconque des revendications précédentes.

13. Procédé de préparation de la composition selon les revendications 1 à 11, le procédé comprenant les étapes suivantes
(a) mélanger le principe actif et le support hydrosoluble pour former un mélange de poudres ou de granules, puis
(b) mélanger le mélange, obtenu à l'étape (a), au liquide huileux.

14. Procédé de préparation de la composition selon les revendications 1 à 11, comprenant les étapes suivantes
(a) disperser ou dissoudre le principe actif dans le liquide huileux pour former une solution, une dispersion ou une suspension liquid, puis
(b) mélanger le support hydrosoluble à la solution, la dispersion ou la suspension liquide obtenue à l'étape (a).

15. Procédé de préparation de la composition selon les revendications 1 à 11, comprenant les étapes suivantes
(a) mélanger le support hydrosoluble et le liquide huileux de manière à obtenir un mélange de poudres ou de granules ; puis
(b) mélanger le mélange, obtenu à l'étape (a), au principe actif.

16. Procédé selon l'une quelconque des revendications 13 à 15, l'étape (a) étant réalisée sans l'aide de solvants ni l'ajout d'excipients liquides supplémentaires.
